# EUROPEAN PATENT APPLICATION

(11) **EP 4 742 261 A1**
(43) Date of publication of application: **13.05.2026**
(21) Application number: 23748824.2
(22) Date of filing: 04.07.2023
(51) Int. Cl.: G16H 20/40, G16H 50/30

(54) **SYSTEM FOR DETECTING A POST-OPERATIVE INFECTION RISK IN AN OPERATING ROOM**

(71) Applicant: Piveu Medtech Solutions, S.L., 08030 Barcelona (ES)
(72) Inventor: DIXON, James, 08030 BARCELONA (ES)
(74) Representative: Herrero & Asociados, S.L.
(86) International application number: PCT/ES2023/070429
(87) International publication number: WO 2025/008551

(57) **Abstract**

System (100) for detecting a post-operative infection risk in an operating room (1000), the system (100) comprising a first sensor (110) configured to identify a sterile field area (120) in the operating room (1000), computing means (110) configured to detect a sterile field occupancy levels within the sterile field area (120), provide a first real-time data (110a) based on the sterile field occupancy, the first real-time data (110a) comprising values between [0-10] users and detect the post-operative infection risk based at least on the first real-time data (110a) comprising a value of at least 5 users, provide an infection risk alarm (130) based on the detection of the post-operative infection risk and display means (140) configured to display at least the first real-time data (110a) and the infection risk alarm (130).

## Description

The present invention refers to a system for detecting a post-operative infection risk in an operating room, OR. The system is configured to collect safety related workflow information from one or more sensors in the operating room, OR.

The technical field falls in the healthcare sector, for hospitals that do surgery, addressing the need for a reduction in post-operative infections, reducing the risk of their development by facilitating safer surgery.

### BACKGROUND OF THE INVENTION

Two hallmarks of modern surgery that have done much to reduce post-operative infections are the creation of the aseptic environment, which is the area near the surgical wound where all surfaces are aseptic, including robing team members in aseptic clothing (commonly called the "sterile field") and the establishment of evidence-based "best practices" for safe workflows both in and out of the sterile field that minimize infection.

However, the implementation of this "evidence-based practice" has unresolved issues in daily practice which have left the post-operative infection rates unacceptably high. Furthermore, the lack of hard-data and the resulting inability to measure and control the level of implementation has led to an unacceptable and wide variability in adherence to these widely accepted best practices and subsequent unacceptably wide variability in post-operative infection rates.

### Pure air in the sterile field or safer workflow

The theory behind the creation of the sterile field is to only allow aseptic elements (instruments and surfaces) close to the patient so no pathogen can enter the wound by touch. Surfaces are draped, staff in the field are robed with aseptic clothing, masked and double gloved; instruments are all sterilized.

However, pathogens can be air-borne. In an attempt to reduce this risk, laminar flow systems have been installed since the 1960s. They release medical grade air over an approximately 3m x 3m area over the patient to create a positive pressure "aseptic air" environment in the sterile area. In recent studies, laminar flow systems have not been shown to reduce post-operatory infections. Laminar air systems have not solved the problem, likely in part due to "turbulence", when workflow causes movement in the OR, causing the mixing of "normal" air in the non-sterile area with the aseptic air over the patient.

Air purifying systems (popularized during the pandemic) have been introduced into the OR as a way of mitigating the risk of turbulence by reducing the number of pathogens in the "normal" air in the OR which can be mixed with the medical grade air through turbulence. The air purifiers are not, however, designed to deal with the "peak" slough off of pathogens at the specific place and time when the aseptic person or device moves. The location and intensity of the "slough off" may be much more relevant as a contamination factor than the levels of air purity in general in the OR. The clinical efficiency of air purifying systems is currently under evaluation.

The practice of measuring of air purity in the OR and correlating it with specific workflows/events during surgery has been used in studies and in practice as a tool for "troubleshooting" ORs with high rates of infection and for teams proactively developing safer workflows. The air purity data is used as an indicator, a proxy of less/more contaminating workflows observed at the time, that the team can then incorporate or eliminate. This method has been successful in helping specific teams proactively seeking to do safer surgery and has contributed to the understanding of what is best practice. However, it has not shown to be a scalable solution capable of making a broad impact on post-operative infection rates in the healthcare system.

Methods accepted by the healthcare community for measuring air purity in the OR environment include the traditional method of taking air samplings of the room and doing the corresponding microbiological testing, laser-detection airborne particle counting or live airborne partial counting (in process of clinical validation).

### Workflows in the OR that increase infection risk

Where an event occurs in relation to the sterile field is a key factor for understanding the risk inherent in the event. For example, any time there is a "violation of the sterile field", the contact any non-aseptic element with the sterile field, the surgery is required to immediately stop and start over after re-establishing the aseptic field. This is more theory than practice. Minor violations are often not observed; the focus of the operating team is on the surgery. And in the case a minor violation does come to the attention of the team, a subjective, largely intuitive, a decision is made about the risk-benefit of restarting the surgery based on the fleeting observation of the event. In practice, minor violations of the sterile field are much more common (albeit unmeasured) then the restarting of a surgery to re-establish the aseptic field. Another example of an important and common risk factor is the presence and movement of inherently non-aseptic elements within and close to the sterile field (essential infrastructure, un-covered staff skin, breath). These events are widely accepted to be risk-factors based on a few studies with very limited data, anecdotal evidence and "common sense". However, the impossibility to date to capture reliable data about these factors has left their importance and frequency in the realm of personal opinion. The presence and movement of not aseptically robed (clean dressed) staff near, or in, the sterile field is also a known factor increasing the risk of infection, The generally accepted "margin of safety" to maintain from the sterile field is 30cm.

However, the only method available for determining the location of the sterile field and the identification of aseptic and non-aseptic elements in relation to that sterile field was the direct, subjective observation of the people in the room. This method has given us limited understanding of these factors and no ability to measure them.

Tracking solutions using radio frequency ID, RFID tags or similar have been designed for tracking the location of staff and equipment in the OR, but do not give information about their (potentially infection risk-increasing) movement while in the OR.

Studies using manual observation have shown that the opening of doors during surgery is a positive risk factor for the later development of infections. Groups of hospitals across the world have initiated awareness and training programs to "keep the doors closed during surgery" with varied adherence over hospitals and time. The technology to measure the opening and closing of doors is common and we are aware of at least one solution that uses that technology to measure door openings in the OR, zNav from Ze-con in Sweden.

The present invention overcomes the aforementioned limitations and drawbacks.

### DESCRIPTION OF THE INVENTION

The present invention provides a system for detecting a post-operative infection risk in an operating room. The system is configured to collect safety related workflow information from various sensors in the OR.

The system can be implemented in the OR, where the information is fed back to the team in real time, but it is also available after the surgery is over as hard data for analysis by the surgical team and other health professionals working to reduce post-operative infection.

The proposed system is a tool for surgical teams to help them implement evidence-based best practice for the reduction of post-operative infection risk in the OR. The solution digitally measures the team's relevant workflows, gives them actionable real-time feedback and keeps the data for their analysis after the surgery, leading to a virtuous cycle of continual improvement based on hard-data, automatically captured. The relevance and specificity of the data for the team is dramatically improved by digitally defining the sterile field and relating the captured data to it. Being able to locate an event in its relation to the field (in, near or out) is an essential factor for understanding the degree of risk that that event represents.

With the sterile field defined and sensors automatically monitor the relevant workflows, the adherence of the surgery to evidence-based best practice will be accurately monitored and reported to the surgical team. By simply reacting to the feedback offered by the solution, they will naturally, continually improve the safety of their surgery.

The data captured from the various sensors will be made more relevant and useful by being able to relate the data-points to each other. For example, air purity detectors located in the sterile field will detect when turbulence has brought non-aseptic air into the sterile field. Events which may have caused that turbulence, like the entrance of someone into the sterile field or the opening of a door, will also be captured.

New standards for evidence-based best practice can be validated and established with the proposed system. Currently there are events not validated by hard evidence which common sense and anecdotal evidence point as factors increasing the risk of post-operative infection. Lamp movement and talking in the sterile field, for example, are widely thought to be contributors to infection risk. The proposed system will be developed to capture these events and providing the hard-data necessary for a true scientific evaluation of their relevance and their inclusion (or not) into evidence-based best practice.

Hence, the aspect of the present invention relates to a system for detecting a post-operative infection risk in an operating room, the system comprising a first sensor configured to identify a sterile field area in the operating room and computing means configured to detect a sterile field occupancy within the sterile field area, provide a first real-time data based on the sterile field occupancy, wherein the first real-time data comprising values between [0-10] users. The computing means are further configured to detect the post-operative infection risk based at least on the first real-time data comprising a value of at least 5 users, provide an infection risk alarm based on the detection of the post-operative infection risk and display means configured to display at least the first real-time data and the infection risk alarm.

In one example, the system further comprises a database configured to store at least the first real-time data.

In one example, the first sensor comprises a camera.

In one example, the system further comprises a second sensor configured to detect users not dressed aseptically, wherein the computing means are further configured to detect at least one user not dressed aseptically entering into a safety area, wherein the safety area comprises the sterile field area and at least 15 cm of margin around the sterile field area, provide a second real-time data on the detection of users not dressed aseptically, wherein the second real-time data comprising values between [0-99] users not dressed aseptically. The computing means are further configured to detect the post-operative infection risk based on the second real-time data comprising a value of at least 1 user not dressed aseptically within the sterile field or the surrounding safety area, wherein the display means are further configured to display the second real-time data, and wherein the database is further configured to store the second real-time data.

In one example, the second sensor comprises a camera.

In one example, the system further comprises a third sensor configured to detect door opening data in the operating room, the door opening data comprises the number of times at least one door opens, wherein the computing means are further configured to provide a third real-time data based on the door openings data, wherein the third real-time data comprising values between [0-99] door openings. The computing means are further configured to detect the post-operative infection risk based on the third real-time data comprising a value of at least 6 door openings, wherein the display means are further configured to display the third real-time data, and wherein the database is further configured to store the third real-time data. In one example, the third sensor to detect door opening data comprises magnet-based sensor located in the operating room doors or one or more cameras.

In one example, the system further comprises a fourth sensor configured to measure air purity in the sterile field by measuring the Colony-Forming Units per m3 (CFU/m3), wherein the computing means are further configured to provide a fourth real-time data based on the air purity measured, wherein the fourth real-time data comprising values between [0-500] CFU/m3. The computing means are further configured to detect the post-operative infection risk based on the fourth real-time data comprising a value of at least 100 CFU/m3, wherein the display means are further configured to display the fourth real-time data and wherein the database is further configured to store the fourth real-time data.

In one example, the fourth sensor is a particle counter.

In one example, the system further comprises a fifth sensor configured to count the time since the first surgical cut is made in the patient until the last wound has been closed, wherein the computing means are further configured to provide a fifth real-time data based on the time that the patient is open data, wherein the fifth real-time data comprising values between [0-300] minutes. The computing means are further configured to detect the post-operative infection risk based on the fifth real-time data comprising a value at least of 50 minutes, wherein the display means are further configured to display the fifth real-time data, and wherein the database is further configured to store the fifth real-time data.

In one example, the fifth sensor is a timer.

In one example, the system further comprises a sixth sensor configured to detect the movement of surgical lamps in the sterile field that comprises the number of times the surgical lamps move within the sterile field, wherein the computing means are further configured to provide a sixth real-time data based on the movement of surgical lamps, wherein the sixth real-time data comprising values between [0-20] number of times. The computing means are further configured to detect the post-operative infection risk based on the sixth real-time data comprising a value at least 2 number of times, wherein the display means are further configured to display the sixth real-time data, and wherein the database is further configured to store the sixth real-time data.

In one example, the sixth sensor comprises a camera.

In one example, the system further comprises a seventh sensor configured to detect user's occupancy in the operating room comprising the total time the operating room is occupied by users, wherein the computing means are further configured to provide a seventh real-time data based on the user's occupancy in the operating room, wherein the seventh real-time data comprising values between [0-20] number of users. The computing means are further configured to detect the post-operative infection risk based on the seventh real-time data comprising a value at least 8 number of users, wherein the display means are further configured to display the seventh real-time data, and wherein the database is further configured to store the seventh real-time data.

In one example, the seventh sensor comprises a camera.

In one example, the system further comprises an eighth sensor configured to record the speaking in the sterile field, wherein the computing means are further configured to count in minutes a total amount of talking, provide an eight real-time data based on the total amount of talking, wherein the eight real-time data comprising values between [0-150] minutes. The computing means are further configured to detect the post-operative infection risk based on the eight real-time data (180a) comprising a value of at least 5 minutes, wherein the display means are further configured to display the eight real-time data, and wherein the database is further configured to store the eight real-time data.

In one example, the eighth sensor comprises an array of microphones.

In one example, the system further comprises a ninth sensor configured to detect the body temperature of the patient over time, wherein the computing means are further configured to measure the time that the patient is hypothermic comprising a core temperature below 35,5°C, provide a ninth real-time data based on the time that the patient is hypothermic, wherein the ninth real-time data comprising values between [0-300] minutes. The computing means are further configured to detect the post-operative infection risk based on the ninth real-time data comprising a value of at least 5 minutes, wherein the display means are further configured to display the ninth real-time data, and wherein the database is further configured to store the ninth real-time data.

In one example, the ninth sensor comprises a thermometer.

In one example, the system further comprises a tenth sensor configured measure the air purity outside the sterile field by measuring the Colony-Forming Units per m3 (CFU/m3) wherein the computing means are further configured to provide a tenth real-time data based on the air purity outside the sterile field, wherein the tenth real-time data comprising values between [0-500] CFU/m3. The computing means are further configured to detect the post-operative infection risk based on the tenth real-time data comprising a value of at least 200 CFU/m3, wherein the display means are further configured to display the tenth real-time data, and wherein the database is further configured to store the tenth real-time data.

In one example, the tenth sensor is a particle counter.

In one example, the display means comprises a screen. In one example, the screen is configured to display integrated video footage of a surgical procedure in the operating room.

### BRIEF DESCRIPTION OF THE DRAWINGS

For a better understanding the above explanation and for the sole purpose of providing an example, some non-limiting drawings are included that schematically depict a practical embodiment.
Figures 1 shows an example of a system for detecting a post-operative infection risk in an operating room according to the present invention.
Figure 2 shows a sensor configured measure the air purity outside the sterile field by measuring the Colony-Forming Units per m3 (CFU/m3).
Figure 3 shows an OR lamp integrating the sensor shown in figure 2.
Figure 4 shows a user interface representing several real-time workflows obtained by the computing means of the system according to the present invention.
Figure 5 shows the user interface wherein the post-operative infection risk is detected, and the post-operative infection risk alarm is displayed to the user.
Figure 6 shows a screen as part of the system according to the present invention displaying the user interface for showing the data and the post-operative infection risk alarms acquired during surgery for analysis after the surgery is completed.

### DESCRIPTION OF A PREFERRED EMBODIMENT

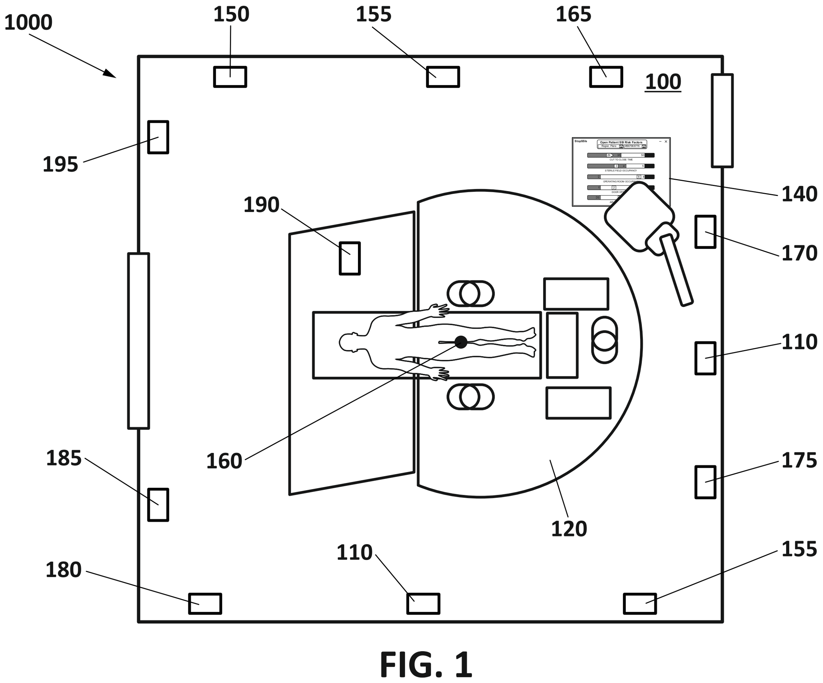

Figure 1 shows an example of a system (100) for detecting a post-operative infection risk in an operating room (1000) according to the present invention, the system (100) comprises computing means, a first sensor (110) configured to identify a sterile field area and the number of people and time that they occupy it (120) in the operating room (1000) and display means (140).

Additionally, the system (100) shown in figure 1 comprises a second sensor (150) configured to detect users not dressed aseptically and their location relative to the sterile field, a third sensor (155) configured to detect door opening data in the operating room, wherein the door opening data comprises the number of times at least one door opens, a fourth sensor (160) configured to measure air purity in the sterile field by measuring the Colony-Forming Units per m3 (CFU/m3), a fifth sensor (165) configured to count the time since the first surgical cut is made in the patient until the last wound has been closed, a sixth sensor (170) configured to detect the movement of surgical lamps in the sterile field that comprises the number of times the surgical lamps move within the sterile field, a seventh sensor (175) configured to detect user's occupancy in the operating room comprising the number of people and time that they occupy the operating room, an eight sensor (180) configured to record the speaking in the sterile field, a ninth sensor (190) configured to detect the body temperature of the patient over time, and a tenth sensor (195) configured measure the air purity outside the sterile field by measuring the Colony-Forming Units per m3 (CFU/m3).

With respect to the first sensor, the computing means (110) are configured to detect a sterile field occupancy within the sterile field area (120), provide a first real-time data (110a) based on the sterile field occupancy, the first real-time data (110a) comprising values between [0-10] users and detect the post-operative infection risk based at least on the first real-time data (110a) comprising a value of at least 5 users, provide an infection risk alarm (130) based on the detection of the post-operative infection risk.

With respect to the second sensor, the computing means (110) are configured to detect at least one user not dressed aseptically entering into a safety area, wherein the safety area comprises the sterile field area (120) and at least 15 cm of margin around the sterile field area (120), provide a second real-time data (150a) based on the detection of users not dressed aseptically, the second real-time data (150a) comprising values between [0-99] users not dressed aseptically entering into the safety area or sterile field and detect the post-operative infection risk based on the second real-time data (150a) comprising a value of at least 1 user not dressed aseptically entering into the safety area or sterile field.

With respect to the third sensor, the computing means (110) are configured to provide a third real-time data (155a) based on the door openings data, the third real-time data comprising values between [0-99] door openings; and detect the post-operative infection risk based on the third real-time data comprising a value of at least 6 door openings.

With respect to the fourth sensor, the computing means (110) are configured to provide a fourth real-time data (160a) based on the air purity in the sterile field measured, the fourth real-time data (160a) comprising values between [0-500] CFU/m3 and detect the post-operative infection risk based on the fourth real-time data (160a) comprising a value of at least 100 CFU/m3.

With respect to the fifth sensor, the computing means (110) are configured to provide a fifth real-time data (165a) based on the cut to close time data, the fifth real-time data (165a) comprising values between [0-300] minutes and detect the post-operative infection risk based on the fifth real-time data (165a) comprising a value at least of 50 minutes.

With respect to the sixth sensor, the computing means (110) are configured to provide a sixth real-time data (170a) based on the movement of surgical lamps, the sixth real-time data (170a) comprising values between [0-20] number of times and detect the post-operative infection risk based on the sixth real-time data (170a) comprising a value at least 2 number of times.

With respect to the seventh sensor the computing means (110) are configured to provide a seventh real-time data (175a) based on the number of people and time that they occupy the operating room, the seventh real-time data (175a) comprising values between [0-20] number of users and detect the post-operative infection risk based on the seventh real-time data (175a) comprising a value at least 8 number of users. The specific values of the ranges can be determined by the hospital. In practice, the hospital can decide on that number of users (e.g., 5, 6 or other), as well as the other ranges relates to other sensors of the present invention as e.g. "safe distance from infection" and "close to infection risk value".

With respect to the eight sensor the computing means (110) are configured to count in minutes a total amount of talking, provide an eight real-time data (180a) based on the total amount of talking, the eight real-time data comprising values between [0-150] minutes; and detect the post-operative infection risk based on the eight real-time data (180a) comprising a value of at least 5 minutes.

With respect to the ninth sensor the computing means (110) are configured to measure the time that the patient is hypothermic comprising a core temperature below 35,5°C, provide a ninth real-time data (190a) based on the time that the patient is hypothermic, the ninth real-time data (190a) comprising values between [0-300] minutes and detect the post-operative infection risk based on the ninth real-time data (190a) comprising a value of at least 5 minutes.

With respect to the tenth sensor the computing means (110) are configured to provide a tenth real-time data (195a) based on the air purity outside the sterile field, the tenth real-time data (195a) comprising values between [0-500] CFU/m3 and detect the post-operative infection risk based on the tenth real-time data (195a) comprising a value of at least 200 CFU/m3.

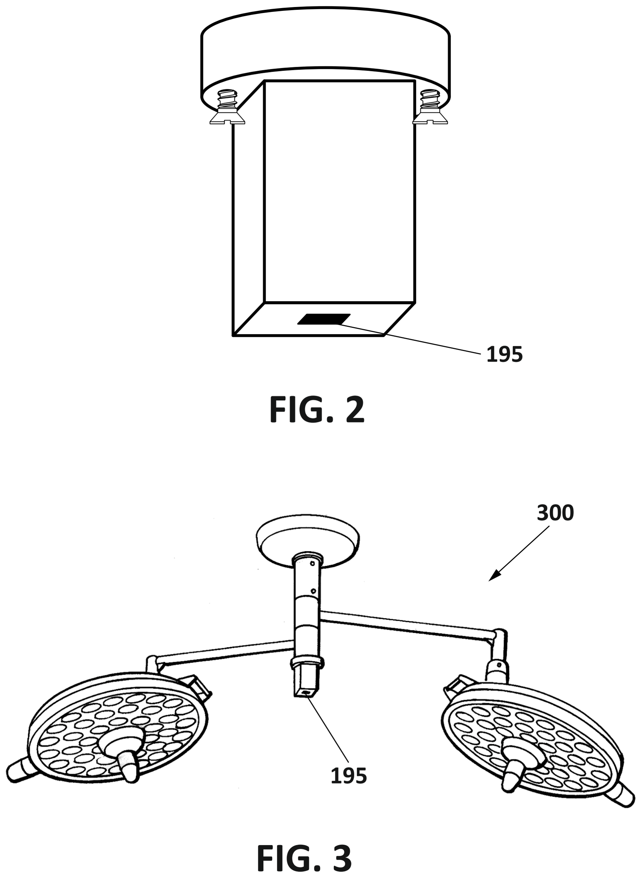

Figure 2 shows the tenth sensor (195) configured measure the air purity in the sterile field by measuring the Colony-Forming Units per m3 (CFU/m3).

Figure 3 shows the tenth sensor (195) attached to the OR lamp (300). As shown in figure 1, the tenth sensor (195) is located on top of the patient, where in the OR lamp (300) is established.

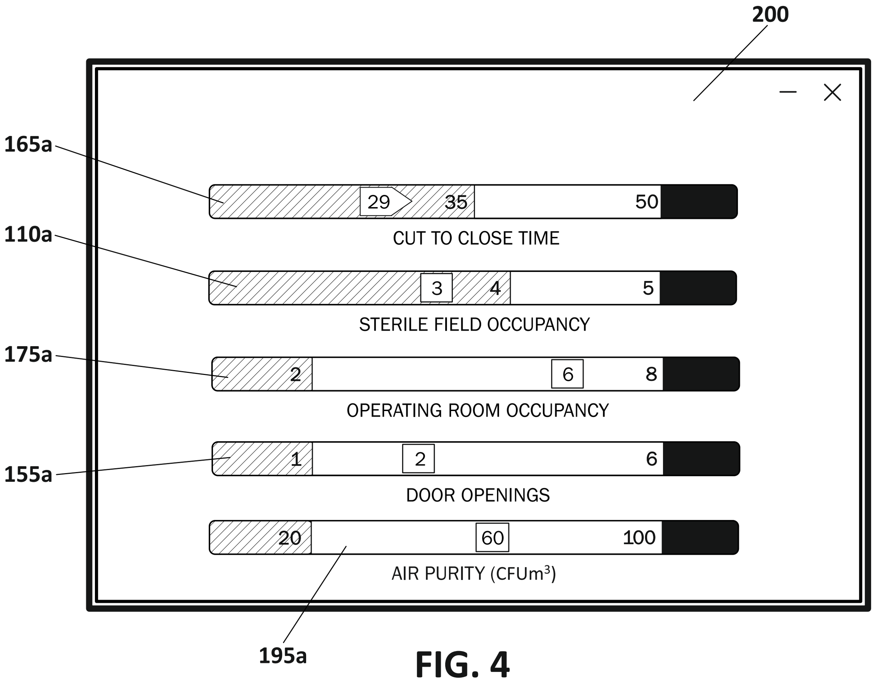

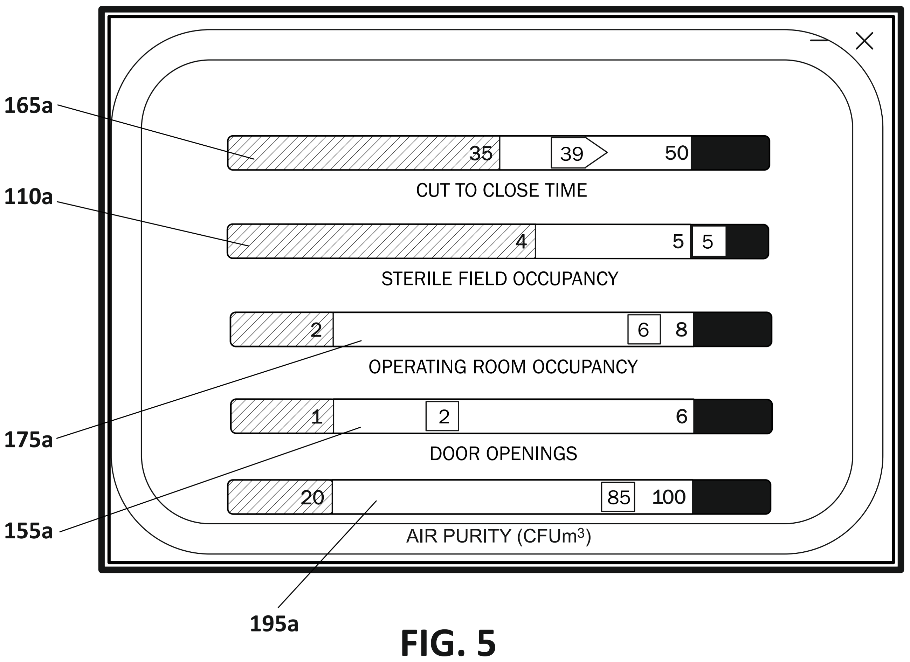

Figure 4 shows the user interface that can be displayed by the display means (140). Feedback about the workflow will be visible to the team in the OR. This user interface is displayed on the display means (140), e.g., a monitor as shown in figure 5, that can be placed outside the sterile field. The display means (140) will show the real-time data with hospital defined "ranges" of when the relevant parameter is considered to be in a safe range, a range close to the infection risk value and a range where the parameter has reached of exceeded the risk detection value. The specific values of the ranges can be determined by the hospital.

In practice, the hospital will decide on that number of users (e.g., 5, 6 or other), as well as the other ranges relates to other sensors of the present invention as e.g. "safe distance from infection" and "close to infection risk value".

In this example, the display means (140) are configured to display from top to the bottom, the fifth real-time data (165a) based on the cut to close time data having a value of 29, wherein the limit to detect the post-operative infection risk is established in 50.

The display means (140) are configured to display the first real-time data (110a) based on the sterile field occupancy having a value of 3, wherein the limit to detect the post-operative infection risk is established in 5.

The display means (140) are configured to display the seventh real-time data (175a) based on the user's occupancy in the operating room having a value of 6, wherein the limit to detect the post-operative infection risk is established in 8.

The display means (140) are configured to display the third real-time data (155a) based on the door openings data, having a value of 2, wherein the limit to detect the post-operative infection risk is established in 6.

The display means (140) are configured to display the tenth real-time data (195a) based on the air purity outside the sterile field having a value of 60, wherein the limit to detect the post-operative infection risk is established in 100.

Figure 5 shows the user interface wherein the post-operative infection risk alarm is detected wherein the fifth real-time data (165a) based on the cut to close time data having a value of 29, the first real-time data (110a) based on the sterile field occupancy having a value of 5, the seventh real-time data (175a) based on the user's occupancy in the operating room having a value of 6, the third real-time data (155a) based on the door openings data, having a value of 2, and the tenth real-time data (195a) based on the air purity outside the sterile field having a value of 85.

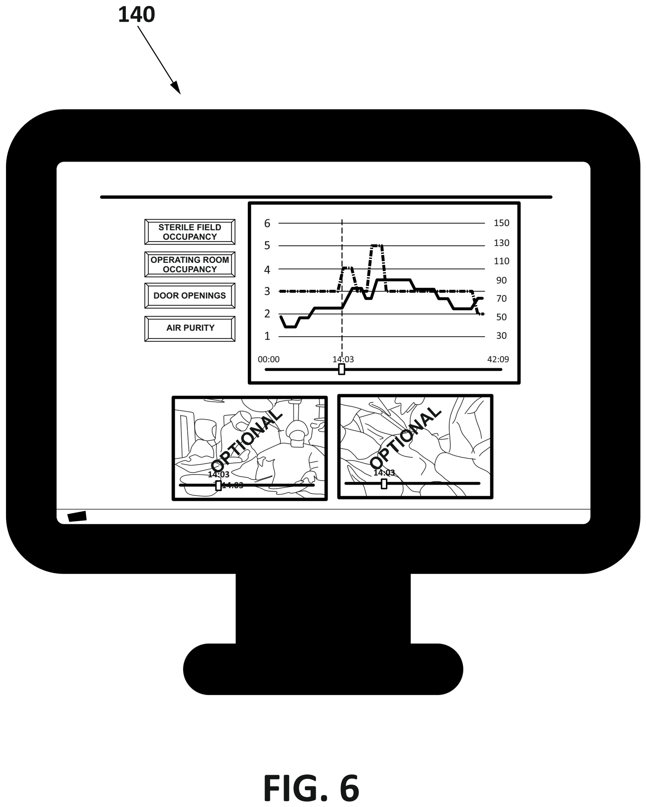

Figure 6 shows the display means being a screen that shows graphical representations of a possible software interface that can be used for post-operation analysis. The data captured during the operating is kept in a database for analysis of workflow and safety evolution during an individual surgery or aggregated data from multiple operations can be looked at in the OR (on the display means (140)) or outside the OR on a network PC or other device. The screen shows an example of a possible software user interface including integrated video footage of the operation.

## Claims

1. System (100) for detecting a post-operative infection risk in an operating room (1000), the system (100) comprising:
- a first sensor (110) configured to identify a sterile field area (120) in the operating room (1000);
- computing means (110) configured to:
detect a sterile field occupancy within the sterile field area (120);
provide a first real-time data (110a) based on the sterile field occupancy, the first real-time data (110a) comprising values between [0-10] users; and
detect the post-operative infection risk based at least on the first real-time data (110a) comprising a value of at least 5 users;
provide an infection risk alarm (130) based on the detection of the post-operative infection risk; and
display means (140) configured to display at least the first real-time data (110a) and the infection risk alarm (130).

2. The system according to claim 1, further comprising a database configured to store at least the first real-time data.

3. The system according to claims 1 or 2, wherein the first sensor (110) comprises a camera.

4. The system according to claims 2 or 3, further comprising:
a second sensor (150) configured to detect users not dressed aseptically,
wherein the computing means (110) are further configured to:
detect at least one user not dressed aseptically entering into a safety area, wherein the safety area comprises the sterile field area (120) and at least 15 cm of margin around the sterile field area (120);
provide a second real-time data (150a) based on the detection of users not dressed aseptically, the second real-time data (150a) comprising values between [0-99] users not dressed aseptically; and
detect the post-operative infection risk based on the second real-time data (150a) comprising a value of at least 1 user not dressed aseptically,
wherein the display means (140) are further configured to display the second real-time data (150a), and
wherein the database is further configured to store the second real-time data (150a).

5. The system according to the previous claim, wherein the second sensor (150) comprises a camera.

6. The system according to claims 2 to 5, further comprising:
- a third sensor (155) configured to detect door opening data in the operating room, the door opening data comprises the number of times at least one door opens;
wherein the computing means (110) are further configured to:
provide a third real-time data (155a) based on the door openings data, the third real-time data comprising values between [0-99] door openings; and
detect the post-operative infection risk based on the third real-time data comprising a value of at least 6 door openings,
wherein the display means (140) are further configured to display the third real-time data (155a), and
wherein the database is further configured to store the third real-time data (155a).

7. The system according to claims 2 to 6, further comprising:
a fourth sensor (160) configured to measure air purity in the sterile field by measuring the Colony-Forming Units per m3 (CFU/m3);
wherein the computing means (110) are further configured to:
provide a fourth real-time data (160a) based on the air purity measured, the fourth real-time data (160a) comprising values between [0-500] CFU/m3; and
detecting the post-operative infection risk based on the fourth real-time data (160a) comprising a value of at least 100 CFU/m3,
wherein the display means (140) are further configured to display the fourth real-time data (160a), and
wherein the database is further configured to store the fourth real-time data (160a).

8. The system according to the previous claim, wherein the fourth sensor (160) is a particle counter.

9. The system according to claims 2 to 5, further comprising:
a fifth sensor (165) configured to count the time since the first surgical cut is made in the patient until the last wound has been closed,
wherein the computing means (110) are further configured to:
provide a fifth real-time data (165a) based on the cut to close time data, the fifth real-time data (165a) comprising values between [0-300] minutes; and
detecting the post-operative infection risk based on the fifth real-time data (165a) comprising a value at least of 50 minutes,
wherein the display means (140) are further configured to display the fifth real-time data (165a), and
wherein the database is further configured to store the fifth real-time data (165a).

10. The system according to the previous claim, wherein the fifth sensor (165) is a timer.

11. The system according to claims 2 to 10, further comprising:
- a sixth sensor (170) configured to detect the movement of surgical lamps in the sterile field that comprises the number of times the surgical lamps move within the sterile field;
wherein the computing means (110) are further configured to:
provide a sixth real-time data (170a) based on the movement of surgical lamps, the sixth real-time data (170a) comprising values between [0-20] number of times; and
detect the post-operative infection risk based on the sixth real-time data (170a) comprising a value at least 2 number of times,
wherein the display means (140) are further configured to display the sixth real-time data (170a), and
wherein the database is further configured to store the sixth real-time data (170a).

12. The system according to claims 2 to 11, further comprising:
- a seventh sensor (175) configured to detect user's occupancy in the operating room comprising the total time the operating room is occupied by users;
wherein the computing means (110) are further configured to:
provide a seventh real-time data (175a) based on the user's occupancy in the operating room, the seventh real-time data (175a) comprising values between [0-20] number of users; and
detect the post-operative infection risk based on the seventh real-time data (175a) comprising a value at least 8 number of users,
wherein the display means (140) are further configured to display the seventh real-time data (175a), and
wherein the database is further configured to store the seventh real-time data (175a).

13. The system according to claims 2 to 12, further comprising:
- an eight sensor (180) configured to record the speaking in the sterile field; wherein the computing means (110) are further configured to:
count in minutes a total amount of talking;
provide an eight real-time data (180a) based on the total amount of talking, the eight real-time data comprising values between [0-150] minutes; and
detect the post-operative infection risk based on the eight real-time data (180a) comprising a value of at least 5 minutes,
wherein the display means (140) are further configured to display the eight real-time data (180a), and
wherein the database is further configured to store the eight real-time data (180a).

14. The system according to claims 2 to 13, further comprising:
- a ninth sensor (190) configured to detect the body temperature of the patient over time;
wherein the computing means (110) are further configured to:
measure the time that the patient is hypothermic comprising a core temperature below 35,5°C;
provide a ninth real-time data (190a) based on the time that the patient is hypothermic, the ninth real-time data (190a) comprising values between [0-300] minutes; and
detect the post-operative infection risk based on the ninth real-time data (190a) comprising a value of at least 5 minutes,
wherein the display means (140) are further configured to display the ninth real-time data (190a), and
wherein the database is further configured to store the ninth real-time data (190a).

15. The system according to claims 2 to 14, further comprising:
- a tenth sensor (195) configured measure the air purity outside the sterile field by measuring the Colony-Forming Units per m3 (CFU/m3)
wherein the computing means (110) are further configured to:
provide a tenth real-time data (195a) based on the air purity outside the sterile field, the tenth real-time data (195a) comprising values between [0-500] CFU/m3; and
detect the post-operative infection risk based on the tenth real-time data (195a) comprising a value of at least 200 CFU/m3,
wherein the display means (140) are further configured to display the tenth real-time data (195a), and
wherein the database is further configured to store the tenth real-time data (195a).

16. The system according to the previous claim, wherein the tenth sensor (195) is a particle counter.

17. The system according to any of the previous claims, wherein the display means (140) comprises a screen.

18. The system according to the previous claim, wherein the screen is configured to display integrated video footage of a surgical procedure in the operating room.

## Amended claims

### Amended claims under Art. 19.1 PCT

1. System (100) for detecting a post-operative infection risk in an operating room (1000), the system (100) comprising:
- a first sensor (110) configured to identify a sterile field area (120) in the operating room (1000);
- computing means (110) configured to:
detect a sterile field occupancy within the sterile field area (120);
provide a first real-time data (110a) based on the sterile field occupancy, the first real-time data (110a) comprising values between [0-10] users; and
detect the post-operative infection risk based at least on the first real-time data (110a) comprising a value of at least 5 users;
provide an infection risk alarm (130) based on the detection of the post-operative infection risk; and
display means (140) configured to display at least the first real-time data (110a) and the infection risk alarm (130);
a database configured to store at least the first real-time data; and
a second sensor (150) configured to detect users not dressed aseptically, wherein the computing means (110) are further configured to:
detect at least one user not dressed aseptically entering into a safety area, wherein the safety area comprises the sterile field area (120) and at least 15 cm of margin around the sterile field area (120);
provide a second real-time data (150a) based on the detection of users not dressed aseptically, the second real-time data (150a) comprising values between [0-99] users not dressed aseptically; and
detect the post-operative infection risk based on the second real-time data (150a) comprising a value of at least 1 user not dressed aseptically,
wherein the display means (140) are further configured to display the second real-time data (150a), and
wherein the database is further configured to store the second real-time data (150a).

2. The system according to claim 1, wherein the first sensor (110) comprises a camera.

3. The system according to the claim 1, wherein the second sensor (150) comprises a camera.

4. The system according to claims 1 to 3, further comprising:
- a third sensor (155) configured to detect door opening data in the operating room, the door opening data comprises the number of times at least one door opens;
wherein the computing means (110) are further configured to:
provide a third real-time data (155a) based on the door openings data, the third real-time data comprising values between [0-99] door openings; and
detect the post-operative infection risk based on the third real-time data comprising a value of at least 6 door openings,
wherein the display means (140) are further configured to display the third real-time data (155a), and
wherein the database is further configured to store the third real-time data (155a).

5. The system according to claims 1 to 4, further comprising:
a fourth sensor (160) configured to measure air purity in the sterile field by measuring the Colony-Forming Units per m3 (CFU/m3);
wherein the computing means (110) are further configured to:
provide a fourth real-time data (160a) based on the air purity measured, the fourth real-time data (160a) comprising values between [0-500] CFU/m3; and
detecting the post-operative infection risk based on the fourth real-time data (160a) comprising a value of at least 100 CFU/m3,
wherein the display means (140) are further configured to display the fourth real-time data (160a), and
wherein the database is further configured to store the fourth real-time data (160a).

6. The system according to the previous claim, wherein the fourth sensor (160) is a particle counter.

7. The system according to claims 1 to 3, further comprising:
a fifth sensor (165) configured to count the time since the first surgical cut is made in the patient until the last wound has been closed,
wherein the computing means (110) are further configured to:
provide a fifth real-time data (165a) based on the cut to close time data, the fifth real-time data (165a) comprising values between [0-300] minutes; and
detecting the post-operative infection risk based on the fifth real-time data (165a) comprising a value at least of 50 minutes,
wherein the display means (140) are further configured to display the fifth real-time data (165a), and
wherein the database is further configured to store the fifth real-time data (165a).

8. The system according to the previous claim, wherein the fifth sensor (165) is a timer.

9. The system according to claims 1 to 8, further comprising:
- a sixth sensor (170) configured to detect the movement of surgical lamps in the sterile field that comprises the number of times the surgical lamps move within the sterile field;
wherein the computing means (110) are further configured to:
provide a sixth real-time data (170a) based on the movement of surgical lamps, the sixth real-time data (170a) comprising values between [0-20] number of times; and
detect the post-operative infection risk based on the sixth real-time data (170a) comprising a value at least 2 number of times,
wherein the display means (140) are further configured to display the sixth real-time data (170a), and
wherein the database is further configured to store the sixth real-time data (170a).

10. The system according to claims 1 to 9, further comprising:
- a seventh sensor (175) configured to detect user's occupancy in the operating room comprising the total time the operating room is occupied by users;
wherein the computing means (110) are further configured to:
provide a seventh real-time data (175a) based on the user's occupancy in the operating room, the seventh real-time data (175a) comprising values between [0-20] number of users; and
detect the post-operative infection risk based on the seventh real-time data (175a) comprising a value at least 8 number of users,
wherein the display means (140) are further configured to display the seventh real-time data (175a), and
wherein the database is further configured to store the seventh real-time data (175a).

11. The system according to claims 1 to 10, further comprising:
- an eight sensor (180) configured to record the speaking in the sterile field; wherein the computing means (110) are further configured to:
count in minutes a total amount of talking;
provide an eight real-time data (180a) based on the total amount of talking, the eight real-time data comprising values between [0-150] minutes; and
detect the post-operative infection risk based on the eight real-time data (180a) comprising a value of at least 5 minutes,
wherein the display means (140) are further configured to display the eight real-time data (180a), and
wherein the database is further configured to store the eight real-time data (180a).

12. The system according to claims 1 to 11, further comprising:
- a ninth sensor (190) configured to detect the body temperature of the patient over time;
wherein the computing means (110) are further configured to:
measure the time that the patient is hypothermic comprising a core temperature below 35,5°C;
provide a ninth real-time data (190a) based on the time that the patient is hypothermic, the ninth real-time data (190a) comprising values between [0-300] minutes; and
detect the post-operative infection risk based on the ninth real-time data (190a) comprising a value of at least 5 minutes,
wherein the display means (140) are further configured to display the ninth real-time data (190a), and
wherein the database is further configured to store the ninth real-time data (190a).

13. The system according to claims 1 to 12, further comprising:
- a tenth sensor (195) configured measure the air purity outside the sterile field by measuring the Colony-Forming Units per m3 (CFU/m3)
wherein the computing means (110) are further configured to:
provide a tenth real-time data (195a) based on the air purity outside the sterile field, the tenth real-time data (195a) comprising values between [0-500] CFU/m3; and
detect the post-operative infection risk based on the tenth real-time data (195a) comprising a value of at least 200 CFU/m3,
wherein the display means (140) are further configured to display the tenth real-time data (195a), and
wherein the database is further configured to store the tenth real-time data (195a).

14. The system according to the previous claim, wherein the tenth sensor (195) is a particle counter.

15. The system according to any of the previous claims, wherein the display means (140) comprises a screen.

16. The system according to the previous claim, wherein the screen is configured to display integrated video footage of a surgical procedure in the operating room.
